# EUROPÄISCHE PATENTSCHRIFT

(11) **EP 0 610 819 B1**
(45) Veröffentlichungstag und Bekanntmachung des Hinweises auf die Patenterteilung: **21.08.1996**
(21) Anmeldenummer: 94101683.4
(22) Anmeldetag: 04.02.1994
(51) Int. Cl.: C07C 45/67, C07C 49/293

(54) **Verfahren zur Herstellung von Acylcyclopropanen**
Process for the preparation of acylcyclopropanes
Procédé pour la préparation d'acylcyclopropanes

(30) Priorität: 11.02.1993 DE 4304003
(43) Veröffentlichungstag der Anmeldung: 17.08.1994
(73) Patentinhaber: BASF Aktiengesellschaft, 67063 Ludwigshafen (DE)
(72) Erfinder: Northemann, Andreas, Dr., D-67071 Ludwigshafen (DE)

(56) Entgegenhaltungen:
- CHEMISTRY LETTERS. 1975 , TOKYO JP Seiten 1149 - 1152 M.ASAOKA ET AL. 'Alkylation Reaction accompanied by Dealkoxycarbonylation of beta - Keto Esters, Geminal Diesters and alpha - Cyano Ester in Heyamethylphosphoric Triamide. (HMPA)'
- TETRAHEDRON LETTERS. Nr. 49 , 1975 , OXFORD GB Seiten 4389 - 4392 S. TAKEI ET AL. 'A novel Synthesis of Cyclopropyl Ketones via Decarboxylative Ring Contractions of alpha - Acyl - gamma - Butyrolactones Catalyzed by Halide Ions in Diploair Aprotic Solvents.'

## Beschreibung

Die vorliegende Erfindung betrifft ein neues Verfahren zur Herstellung von Acylcyclopropanen der allgemeinen Formel I 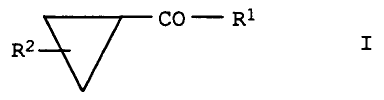
in der R¹ einen aliphatischen Rest mit 1 bis 4 C-Atomen und R² einen der Reste R¹ oder Wasserstoff bezeichnen, unter Verwendung von Katalysatoren, die für diese Reaktion an sich bekannt sind.

Die Verbindungen I sind bekannt und dienen als Zwischenprodukte für die Synthese von Pflanzenschutzmitteln und Pharmaprodukten.

Die Decarboxylierung von 3-Acyl-tetrahydrofuran-2-onen II zu Acylcyclopropanen I mit Hilfe eines Alkalimetallsalzes als Katalysator in homogener flüssiger Phase in Gegenwart eines hochsiedenden Lösungsmittels wie Dimethylformamid, Dimethylsulfoxid, N-Methyl-pyrrolidon oder Hexamethylphosphorsäuretriamid ist aus Synth. Commun. 16 (7), 837 (1986) und Synthesis 1982, 909 bekannt. Gute Ausbeuten werden nur bei Verwendung von Hexamethylphosphorsäuretriamid erhalten, welches jedoch teuer und darüber hinaus physiologisch nicht unbedenklich ist.

Der Erfindung lag deshalb die Aufgabe zugrunde, Acylcyclopropane I auf einfachere und wirtschaftlichere Weise zugänglich zu machen.

Demgemäß wurde ein neues Verfahren zur Herstellung von Acylcyclopropanen der allgemeinen Formel I 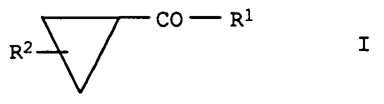
in der R¹ einen aliphatischen Rest mit 1 bis 4 C-Atomen und R² einen der Reste R¹ oder Wasserstoff bezeichnen, unter Verwendung von Katalysatoren, die für diese Reaktion an sich bekannt sind, gefunden, welches dadurch gekennzeichnet ist, daß man ein 3-Acyltetrahydrofuran-2-on der allgemeinen Formel II 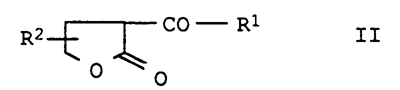
bei erhöhter Temperatur an einem Katalysatorbett in der Gasphase umsetzt.

Das erfindungsgemäße Verfahren läßt sich im einfachsten Falle mit R¹=Me, R²=H und Katalysator = J⁻ wie folgt veranschaulichen:

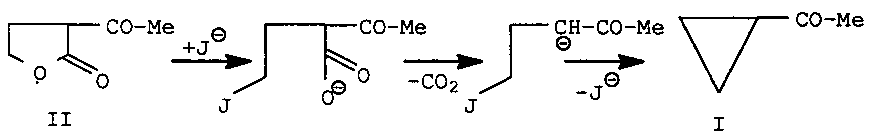

Die Ausgangsverbindungen II sind bekannt oder nach bekannten Methoden erhältlich, z.B. durch Umsetzung von Tetrahydrofuran-2-on mit einem Essigsäurealkylester in Gegenwart eines Natriumalkoholats zum 3-Acyl-tetrahydrofuran-2-on (DE-A 801276) oder durch Reaktion von Acetessigsäureethylester und Ethylenoxid in Gegenwart von Natriumethylat zum 3-Acetyl-tetrahydrofuran-2-on (US-A 2443827).

Unter den definitionsgemäßen Verbindungen II werden aus wirtschaftlichen Gründen solche bevorzugt, in denen R¹ und R² als aliphatische Reste C₁-C₄-Alkylgruppen wie Methyl, Ethyl, n-Propyl, iso-Propyl, n-Butyl und iso-Butyl bezeichnen.

Im Hinblick auf die gewünschten Verfahrensprodukte I ist das 3-Acetyl-tetrahydrofuran-2-on besonders bevorzugt.

Die im Verfahren gemäß der Erfindung einzusetzenden Katalysatoren sind für diese Reaktion an sich bekannt. So eignen sich eine Reihe von Metallsalzen, darunter bevorzugt solche der Toluolsulfonsäure und der Schwefelwasserstoffsäure.

Besonders bevorzugt sind Metallsalze der Halogenwasserstoffsäuren, darunter vor allem die des Bromwasserstoffs und des Jodwasserstoffs.

Da das Metall nur einen untergeordneten Einfluß auf die Reaktion hat, kann es in weiten Grenzen variiert werden.

Bevorzugt eignen sich Erdalkalimetalle und Elemente der ersten und zweiten Nebengruppe des Periodensystems wie insbesondere Kupfer und Zink.

Weiterhin kommen Zinn und Seltene Erdmetalle wie Samarium in Betracht. Außerdem eignen sich Ammoniumsalze wie Tetraalkylammoniumhalogenide. Besonders bevorzugt sind Alkalimetallsalze wie Lithiumiodid, Natriumiodid und Kaliumiodid.

Die obigen Katalysatoren können direkt als feste Substanzkatalysatoren oder vorzugsweise als feste Trägerkatalysatoren verwendet werden.

Als Trägermaterialien verwendet man bevorzugt Siliciumoxide, Tonerden und Zeolithe.

Außerdem eignen sich Magnesiumoxid, Lanthanoxid, Zirkonoxid, Bariumsulfat und Aluminiumsilikat.

Besonders bevorzugte Träger sind Aluminiumoxid und vor allem Zinkoxid und Titandioxid.

Die Herstellung des Trägerkatalysators erfolgt üblicherweise durch Tränken oder durch Besprühen des Trägermaterials mit einer das Salz enthaltenen Lösung sowie anschließendes Trocknen.

Die Trägerkatalysatoren haben vorzugsweise einen Gehalt an aktiven Salzen von 0,5 bis 25, besonders 1 bis 10 Gew.-%.

Die Katalysatoren können als 2- bis 4-mm-Stränge, als Tabletten mit 3 bis 5 mm Durchmesser, als Kugeln mit einem Durchmesser von 1 bis 10 mm, als Splitt mit Teilchengrößen von 0,05 bis 1 mm oder als Pulver mit Teilchengrößen von 0,05 bis 0,5 mm eingesetzt werden, wobei sich das Pulver auch als Wirbelkontakt eignet. Besonders bevorzugt sind Katalysatoren in Form von Strängen.

Die Katalysatorbelastung beträgt beim kontinuierlichen Verfahren im allgemeinen 0,01 bis 10 g, insbesondere 0,05 bis 2 g der Verbindung II pro g Katalysator und Stunde.

Da in der Gasphase umgesetzt wird, muß die Temperatur mindestens so hoch gewählt werden, daß ein vollständiges Verdampfen der Ausgangsverbindung II gewährleistet ist. Üblicherweise führt man daher die Reaktion bei 220 bis 500°C, vorzugsweise bei 250 bis 350°C durch.

Zweckmäßigerweise wird die Umsetzung bei Normaldruck oder im Falle hochsiedener Ausgangsverbindungen bei vermindertem Druck vorgenommen.

Die Verweilzeiten des Gases im Reaktionsraum betragen normalerweise 0,1 bis 30 Sekunden, meistens 0,5 bis 20 Sekunden.

Es empfiehlt sich, die Ausgangsverbindung II nicht in reiner Form, sondern in Mischung mit einem Inertgas im Volumenverhältnis des Inertgases zur Verbindung II von bevorzugt 1000 : 1 bis 5 : 1, besonders 500 : 1 bis 10 : 1 einzusetzen. Bevorzugt sind Kohlendioxid, Edelgase und Wasserstoff. Besonders bevorzugt wird Stickstoff als Inertgas verwendet.

Die Decarboxylierung erfolgt in der Gasphase, wobei eine Festbettreaktion und eine Gasphasenreaktion im Wirbelbett in Betracht kommen.

Eine wirtschaftlich sehr vorteilhafte Ausführungsform des erfindungsgemäßen Verfahrens besteht in der Durchführung an einem festen Katalysatorbett, indem man das 3-Acyl-tetrahydrofuran-2-on verdampft und als Gasphase, bevorzugt im Gemisch mit einem Inertgas, über einen festen Katalysator leitet.

Die Aufarbeitung des Reaktionsgemisches auf die Verfahrensprodukte I erfolgt in an sich bekannter Weise, und zwar in der Regel durch Destillation oder Rektifikation der kondensierten Reaktionsausträge.

Die nach dem erfindungsgemäßen Verfahren auf einfache und wirtschaftliche Weise erhältlichen Acylcyclopropane I dienen als Zwischenprodukte zur Synthese von Pflanzenschutzmitteln und Pharmaprodukten. So kann man beispielsweise durch Umsetzung von Cyclopropylmethylketon I mit Essigsäuremethylester in einer Esterkondensation das 1-Cyclopropyl-1,3-butandion herstellen, welches seinerseits zur Herstellung von Pflanzenschutzmitteln weiterverwendet wird. Die Reaktion von Cyclopropylmethylketon I mit Hydroxylamin zum Oxim und dessen weitere Umsetzung mit Schwefelsäure führt in einer Beckmann-Umlagerung zum Cyclopropylamin, welches ebenso der Pflanzenschutzmittelherstellung dient.

### Beispiele

### 1. Herstellung der Trägerkatalysatoren

a g Träger T in der Form F wurden 4 Stunden bei 150°C im Vakuum getrocknet. Nach der Durchmischung mit einer Lösung aus 5,1 g Lithiumiodid in 150 ml Wasser sowie Stehenlassen dieser Mischung für die Dauer von 5 Stunden bei Raumtemperatur wurde der Trägerkatalysator getrocknet, indem man zunächst überstehendes Wasser abdekantierte und anschließend den Katalysator 12 Stunden bei 150°C im Vakuum trocknete.

Der Trägerkatalysator enthielt b Gew.-% Iodid, bezogen auf die Summe aus Träger und Iodid.

Die Einzelheiten dieser Versuche sowie deren Ergebnisse sind der Tabelle 1 zu entnehmen.

**Tabelle 1**

| Herstellung der Trägerkatalysatoren A bis D | | | | |
|---|---|---|---|---|
| Bsp. | a (g) | T | F | b (Gew.-%) |
| A | 127 | SiO₂ | 4-mm-Stränge | 1,4 |
| B | 100 | ZnO | 4-mm-Stränge | 3,4 |
| C | 100 | Al₂O₃ | 3-mm-Kugeln | 1,7 |
| D | 100 | TiO₂ | 4-mm-Stränge | 3,2 |

### 2. Herstellung von Cyclopropylmethylketon I

Innerhalb einer Stunde wurden 10 g 3-Acetyl-tetra-hydrofuran-2-on II und a 1 Stickstoff bei einer Temperatur von T°C verdampft und innerhalb von b Sekunden über 100 g eines festen Katalysators K geleitet.

Die kondensierten Reaktionsausträge wurden destilliert und lieferten das Cyclopropylmethylketon I.

Die Einzelheiten dieser Versuche sowie deren Ergebnisse sind der Tabelle 2 zu entnehmen.

**Tabelle 2**

| Herstellung von Cyclopropylenmethylketon I | | | | | |
|---|---|---|---|---|---|
| Bsp. | a(l/h) | T(°C) | b (sec) | K | Ausbeute an I (%) |
| 1 | 100 | 300 | 1,8 | A | 64 |
| 2 | 100 | 300 | 1,8 | B | 72 |
| 3 | 100 | 280 | 1,9 | B | 68 |
| 4 | 100 | 320 | 1,7 | B | 71 |
| 5 | 150 | 320 | 1,2 | B | 81 |
| 6 | 180 | 330 | 1,0 | B | 85 |
| 7 | 100 | 280 | 1,9 | C | 52 |
| 8 | 100 | 300 | 1,8 | D | 68 |
| 9 | 100 | 340 | 1,7 | D | 52 |
| 10 | 200 | 340 | 0,8 | D | 76 |

In einem Dauerversuch analog Beispiel 6 über 200 Stunden zeigte sich, daß die Aktivität des Katalysators erhalten blieb.

## Patentansprüche

1. Verfahren zur Herstellung von Acylcyclopropanen der allgemeinen Formel I 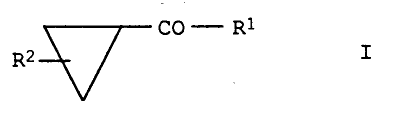 in der R¹ einen aliphatischen Rest mit 1 bis 4 C-Atomen und R² einen der Reste R¹ oder Wasserstoff bezeichnen, unter Verwendung von Katalysatoren, die für diese Reaktion an sich bekannt sind, dadurch gekennzeichnet, daß man ein 3-Acyl-tetrahydrofuran-2-on der allgemeinen Formel II 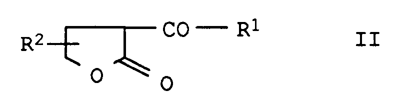 bei erhöhter Temperatur an einem Katalysatorbett in der Gasphase umsetzt.

2. Verfahren nach Anspruch 1, dadurch gekennzeichnet, daß man als Katalysatoren Salze von Halogenwasserstoffsäuren auf festen Trägern verwendet.

3. Verfahren nach Anspruch 2, dadurch gekennzeichnet, daß man als Katalysatoren Salze des Bromwasserstoffs oder Iodwasserstoffs auf festen Trägern verwendet.

4. Verfahren nach den Ansprüchen 1 bis 3, dadurch gekennzeichnet, daß man Zinkoxid oder Titandioxid als festen Träger verwendet.

## Claims

1. A process for the preparation of acylcyclopropanes of the general formula I 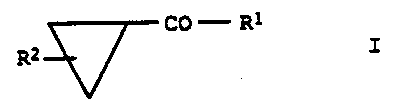 where R¹ is an aliphatic radical having 1 to 4 C atoms and R² is one of the radicals R¹ or hydrogen, using catalysts which are known per se for this reaction, which comprises reacting a 3-acyltetrahydrofuran-2-one of the general formula II 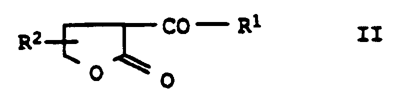 in the gas phase at elevated temperature on a catalyst bed.

2. A process as claimed in claim 1, wherein the catalysts used are salts of hydrohalic acids on solid supports.

3. A process as claimed in claim 2, wherein the catalysts used are salts of hydrogen bromide or hydrogen iodide on solid supports.

4. A process as claimed in claims 1 to 3, wherein zinc oxide or titanium dioxide is used as solid support.

## Revendications

1. Procédé de préparation d'acylcyclopropanes de formule générale I 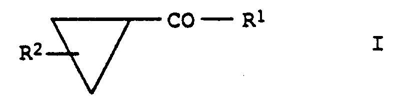 dans laquelle R¹ représente un reste aliphatique à 1-4 atomes de carbone et R² représente l'un des restes R¹ ou un atome d'hydrogène, avec utilisation de catalyseurs qui sont en soi connus pour cette réaction, caractérisé en ce que l'on fait réagir une 3-acyltétrahydrofuranne-2-one de formule générale II 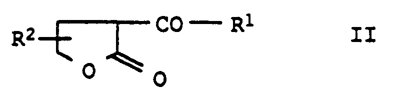 en phase gazeuse, à température élevée, sur un lit de catalyseur.

2. Procédé selon la revendication 1, caractérisé en ce que l'on utilise, comme catalyseurs, des sels d'hydracides halogénés sur des supports solides.

3. Procédé selon la revendication 2, caractérisé en ce que l'on utilise, comme catalyseurs, des sels de l'acide bromhydrique ou de l'acide iodhydrique sur des supports solides.

4. Procédé selon l'une quelconque des revendications 1 à 3, caractérisé en ce que l'on utilise de l'oxyde de zinc ou du dioxyde de titane comme support solide.
